# EUROPEAN PATENT SPECIFICATION

(11) **EP 3 656 865 B1**
(45) Date of publication and mention of the grant of the patent: **12.10.2022**
(21) Application number: 18208026.7
(22) Date of filing: 23.11.2018
(51) Int. Cl.: C12P 1/00, C12P 19/04, C12P 19/02, C12M 1/00

(54) **METHOD FOR COOLING BIOMASS**
VERFAHREN ZUR KÜHLUNG VON BIOMASSE
PROCÉDÉ DE REFROIDISSEMENT DE BIOMASSE

(43) Date of publication of application: 27.05.2020
(73) Proprietor: VALMET AB, 851 94 Sundsvall (SE)
(72) Inventor: Pettersson, Patrik, 865 32 Alnö (SE); Carlsson, Johan, 865 33 Alnö (SE)
(74) Representative: Maiwald Patent- und Rechtsanwaltsgesellschaft mbH

(56) References cited:
- WO-A2-2016/094877
- SONG ET AL: "Process intensification of cellulosic ethanol production by waste heat integration", CHEMICAL ENGINEERING RESEARCH AND DESIGN, vol. 132, 31 January 2018 (2018-01-31), pages 115-122, XP085367985,
- KANG ET AL: "Development and economic analysis of bioethanol production facilities using lignocellulosic biomass", JOURNAL OF BIOSCIENCE AND BIOENGINEERING (ARTICLE IN PRESS), vol. xxx, April 2019 (2019-04), pages 1-5, XP002790921,

## Description

### Field of the invention

The invention generally relates to a treatment of biomass. In particular, the invention relates to a method and a device for treating biomass, wherein the biomass is firstly exposed to a prehydrolysis step, then the biomass treated as such is dewatered and subsequently to an enzymatic hydrolysis step.

### Background art

When treating biomass it is known in the art to firstly pretreat the biomass in a prehydrolysis reactor in which the hemicellulose is dissolved from the biomass under pressure and heat. Depending on the method of discharging the pretreated biomass from the prehydrolysis reactor, e.g. by steam explosion or dilusion discharge, the pretreated biomass has a temperature of about 80 to 200 °C and comprises a certain amount of liquid. For the hydrolysis step the pretreated biomass has to be cooled to a temperature range of optimum enzymatic activity, which is typically about 40 to 60 °C but could be broader depending on the enzyme mixed used. It is known in the art to cool the pretreated biomass by mixing with cold liquid such as cold water. However, for ensuring sufficient hydrolysis activity and further optimizing downstream processing the weight amount of the biomass in the cold biomass slurry is aimed to be adjusted to about 15-25 % preferably 17 to 20 wt%, so that the addition of cold liquid is limited and not sufficient to cool the biomass to the required temperature range. The biomass therefore additionally has to be cooled in a heat exchanger. Heat exchangers have the drawback of low cooling efficiency and can easily plug. Too low weight amounts of biomass in the biomass slurry below 15 wt% have the drawback of suboptimal enzymatic hydrolysis and higher costs because higher amounts of liquid have to be removed from the biomass slurry in the downstream processing.

WO 2016/094877 A2 discloses a process for conversion of pretreated lignocellulosic biomass by enzymatic hydrolysis at a high solids concentration in which the hot pretreated biomass is cooled by a cold stream of aqueous alkaline solution and subsequently dewatered for increasing the concentration of the biomass in the cooled biomass slurry. Said method has the drawback that a high amount of cooling liquid has to be added and a high amount of liquid has to be removed in order to obtain a cooled biomass slurry with a high concentration of biomass, rendering the method ineffective due to high handling costs of the accordant liquid streams.

Therefore, there is a need in the art for an improved method for treating biomass which ensures effective cooling and high concentrations of biomass in the cooled biomass slurry for effective hydrolysis and post-processing.

In the present invention it has been found that by dewatering the thermally treated biomass and cooling the dewatered biomass with a cooling liquid the biomass can be efficiently cooled and the concentration of biomass in the cooled biomass slurry can be effectively adjusted for effective hydrolysis and post-processing.

### Summary of the invention

The present invention relates to a method for treating biomass comprising
(a) Feeding biomass to a pressurized prehydrolysis reactor unit;
(b) Thermally treating the biomass in the pressurized prehydrolysis reactor unit;
(c) Discharging the thermally treated biomass from the pressurized prehydrolysis reactor unit to produce a discharged thermally treated aqueous slurry of biomass;
(d) Dewatering the discharged thermally treated aqueous slurry of biomass;
(e) Feeding the dewatered thermally treated aqueous slurry of biomass into a cooling unit;
(f) Cooling the dewatered thermally treated aqueous slurry of biomass by mixing the dewatered thermally treated aqueous slurry of biomass with a liquid which has a lower temperature than the dewatered thermally treated aqueous slurry of biomass in the cooling unit;
(g) Treating the cooled aqueous slurry of biomass with an enzyme.

Further, the present invention relates to a device for treating biomass comprising
- a biomass feeding system;
- a pressurized prehydrolysis reactor unit for thermally treating biomass and for discharging a thermally treated aqueous slurry of biomass;
- a dewatering unit for separating the discharged thermally treated aqueous slurry of biomass into a dewatered thermally treated aqueous slurry of biomass and a filtrate of hot aqueous solution;
- a cooling unit;
wherein
the biomass feeding system is adapted to feed biomass to the pressurized prehydrolysis reactor unit and to feed the discharged thermally treated aqueous slurry of biomass from the pressurized prehydrolysis reactor unit through the dewatering unit to the cooling unit, and the cooling unit comprises
at least one feeding port for the dewatered thermally treated slurry of biomass;
at least one feeding port for a liquid which has a lower temperature than the dewatered thermally treated aqueous slurry of biomass;
means for mixing the dewatered thermally treated slurry of biomass and the liquid; and at least one feeding port for the enzyme for treating the cooled aqueous slurry of biomass downstream of the at least one feeding port for the dewatered thermally treated slurry of biomass and the at least one feeding port for a liquid which has a lower temperature than the dewatered thermally treated aqueous slurry of biomass; and
at least one discharge port for discharging the enzyme treated slurry of biomass from the cooling unit.

### Definitions

Biomass is any source of plant material suitable for converting into pulp and paper material, cellulose-based construction material or biofuel. Suitable sources of biomass are lignocellulosic biomass such as virgin biomass, waste biomass and energy crops. Virgin biomass includes all naturally occurring terrestrial plants such as trees, bushes and grass. Waste biomass is produced as a low-value byproduct of various industrial sectors such as agriculture, e.g. selected from corn stover, sugarcane bagasse, straw etc., and forestry, e.g. selected from saw mill and paper mill discards. Energy crops are crops with high yield of lignocellulosic biomass produced to serve as a raw material for production of second generation (2G) biofuel. Examples include switch grass (*Panicum virgatum*) and elephant grass.

A filtrate of a hot aqueous solution is an aqueous liquid separated from the thermally treated aqueous slurry of biomass discharged from the pressurized prehydrolysis reactor unit. The aqueous liquid comprises water, and optionally biomass, inorganic substances, such as ash, and water soluble organic substances originating from the thermally treated biomass, such as e.g. hemicellulose, mono- or oligosaccharides.

### Detailed description

### Method

The present invention relates to a method for treating biomass comprising
(a) Feeding biomass to a pressurized prehydrolysis reactor unit;
(b) Thermally treating the biomass in the pressurized prehydrolysis reactor unit;
(c) Discharging the thermally treated biomass from the pressurized prehydrolysis reactor unit to produce a discharged thermally treated aqueous slurry of biomass;
(d) Dewatering the discharged thermally treated aqueous slurry of biomass;
(e) Feeding the dewatered thermally treated aqueous slurry of biomass into a cooling unit;
(f) Cooling the dewatered thermally treated aqueous slurry of biomass by mixing the dewatered thermally treated aqueous slurry of biomass with a liquid which has a lower temperature than the dewatered thermally treated aqueous slurry of biomass in the cooling unit;
(g) Treating the cooled aqueous slurry of biomass with an enzyme.

Before feeding the biomass into the pressurized prehydrolysis reactor the biomass can be pretreated by cutting and/or washing.

The biomass can additionally be pretreated by dilute acid hydrolysis by contacting with a dilute solution containing an external acidic catalyst, reinforced autohydrolysis by contacting with a dilute solution containing a recirculated acidic catalyst or autohydrolysis by contacting with water. These pretreatment measures are well known in the art.

In one embodiment the biomass fed into the pressurized prehydrolysis reactor unit is preferably a solid dry material.

In another embodiment the biomass fed into the pressurized prehydrolysis reactor unit is preferably an aqueous slurry of biomass.

The biomass is fed into the pressurized prehydrolysis reactor unit by means of a feeding system. The feeding system preferably comprises means for actively conveying the biomass into the pressurized prehydrolysis reactor unit such as a screw feeder, e.g. a plug screw feeder optionally with a force feed screw feeding the plug screw feeder.

Suitable means for actively conveying the biomass into the pressurized prehydrolysis reactor unit are known in the art.

The pressurized prehydrolysis reactor unit can comprise one or more such as one or two reactors.

In one embodiment the pressurized prehydrolysis reactor unit comprises more than one reactor such as two reactors. If the pressurized prehydrolysis reactor unit comprises more than one reactor, e.g. an impregnation reactor and a prehydrolysis reactor, the reactors are usually arranged in series. For example, biomass can be fed into an impregnation reactor and then be impregnated in the impregnation reactor. Afterwards, the biomass can be discharged from the impregnation reactor and fed to the subsequent respectively downstream prehydrolysis reactor. The pressurized prehydrolysis reactor unit can also comprise two or more prehydrolysis reactors arranged in series and operating at same or different temperature and pressure.

In another embodiment the pressurized prehydrolysis reactor unit comprises only one reactor which is a prehydrolysis reactor.

In the pressurized prehydrolysis reactor unit the biomass is thermally treated preferably in the presence of water under pressure, more preferably by heating with steam, to produce a thermally treated biomass. In particular, the biomass can be exposed to steam in the pressurized prehydrolysis reactor unit, wherein the steam is fed into the prehydrolysis reactor unit in addition to the biomass. By exposing the biomass to steam, the biomass is pressurized in the prehydrolysis reactor unit, and by heating the pressurized biomass, the thermally treated biomass is produced.

The thermally treated biomass preferably has a temperature of from 50 °C to 250 °C, preferably from 80 °C to 200 °C.

The thermally treated biomass is discharged from the pressurized prehydrolysis reactor unit to produce a discharged thermally treated aqueous slurry of biomass, for example in a blow valve or restriction, which may have the shape of a hole, arranged downstream the pressurized prehydrolysis reactor unit.

Further, the discharged thermally treated aqueous slurry of biomass generally has a weight amount of biomass of from 4 to 45 wt%, preferably of from 8 to 40 wt%, based on the total weight of the discharged thermally treated aqueous slurry of biomass.

In one embodiment the thermally treated biomass is discharged through steam explosion. The steam explosion is defined as a rapid pressure decrease leading to a flashing of the steam. By means of steam explosion the fibers of the biomass disrupted to improve the accessibility of the carbohydrates, such as cellulose and hemicellulose, for subsequent processes, such as enzymatic hydrolysis. Steam explosion is a technique well known in the art of biomass pretreatment.

For steam explosion the biomass is thermally treated by contacting the biomass with steam under elevated pressure in the pressurized reactor.

In this embodiment the thermally treated biomass preferably has a temperature of from 120 °C to 220 °C, more preferably from 170 °C to 210 °C and most preferably from 180 °C to 210 °C.

When discharging the thermally treated biomass through steam explosion the weight amount of the biomass in the discharged thermally treated aqueous slurry of biomass is preferably in the range of from 25 to 60 wt%, more preferably in the range of from 30 to 55 wt%, and most preferably in the range of from 40 to 55 wt%, based on the total weight of the discharged thermally treated aqueous slurry of biomass.

Steam explosion is especially effective for the pretreatment of agricultural residues and hardwood.

In another embodiment the thermally treated biomass is discharged through dilution discharge. For dilution discharge the thermally treated biomass is diluted before it is discharged from the pressurized prehydrolysis reactor unit. In particular, the thermally treated biomass is diluted downstream of the prehydrolysis reactor unit, e.g. between the pressurized prehydrolysis reactor unit and a neutralization unit and a neutralization unit, where the enzyme treatment is applied. The diluted biomass can be discharged e.g. through an orifice and/or a blow valve of the pressurized prehydrolysis reactor unit or can be pumped out of the reactor.

While being discharged, the temperature of the biomass can be between 50 °C to 140 °C, more preferably from 65 °C to 130 °C and most preferably from 80 °C to 120 °C.

In particular, the biomass can have a temperature below 100 °C during discharging and no flashing occurs during discharging. Alternatively, the biomass can have a temperature above 100 °C during discharging and a flashing occurs. However, this flashing occurs without a steam explosion occurring during discharging.

Dilution discharge is a technique well known in the art of biomass pretreatment.

When discharging the thermally treated biomass through dilution discharge the weight amount of the biomass in the discharged thermally treated aqueous slurry of biomass is preferably in the range of from 5 to 25 wt%, more preferably in the range of from 8 to 20 wt%, and most preferably in the range of from 10 to 15 wt%, based on the total weight of the discharged thermally treated aqueous slurry of biomass.

After discharging the thermally treated biomass from the pressurized prehydrolysis reactor unit the discharged thermally treated aqueous slurry of biomass is dewatered.

Generally the weight amount of the biomass in the dewatered thermally treated aqueous slurry of biomass in the range of from 30 to 70 wt%, preferably in the range of from 35 to 65 wt%, based on the total amount of the dewatered thermally treated aqueous slurry of biomass.

After discharge through steam explosion the discharged thermally treated aqueous slurry of biomass is preferably to a weight amount of biomass in the dewatered thermally treated aqueous slurry of biomass of from 30 to 60 wt%, more preferably of from 30 to 55 wt% and most preferably of from 30 to 50 wt%.

After discharge through dilution discharge the discharged thermally treated aqueous slurry of biomass is preferably to a weight amount of biomass in the dewatered thermally treated aqueous slurry of biomass of from 30 to 70 wt%, more preferably of from 35 to 60 wt% and most preferably of from 40 to 60 wt%.

For dewatering the discharged thermally treated aqueous slurry of biomass is preferably fed into a dewatering unit to separate a dewatered thermally treated aqueous slurry of biomass and a filtrate of a hot aqueous solution.

During the dewatering step the temperature of the discharged thermally treated aqueous slurry of biomass is preferably not manipulated so that the dewatered thermally treated aqueous slurry of biomass and the filtrate of a hot aqueous solution preferably are in the same temperature range as the discharged thermally treated aqueous slurry of biomass.

Suitable dewatering units are all units suitable for separating a liquid from a slurry at elevated temperatures, such as e.g. a sieving unit, a pressing unit, a decanting unit such as a decanter centrifuge or a compression unit such as a screw press or a plug screw. Such dewatering units are well known in the art.

The filtrate of hot aqueous solution can be discharged.

However, it is preferred that the filtrate of hot aqueous solution is recycled in the process of the invention.

Thereby, the filtrate of hot aqueous solution can be recycled into the pressurized prehydrolysis reactor unit.

It is, however, preferred that at least part of the filtrate of hot aqueous solution is fed into a heat exchanger to produce a filtrate of a cold aqueous solution.

Preferably, the filtrate of hot aqueous solution is cooled in the heat exchanger to a temperature of 10 to 40 °C, more preferably of 15 to 35 °C.

At least part of the filtrate of cold aqueous solution is fed into the cooling unit of step (f) to cool the dewatered thermally treated aqueous slurry of biomass.

Depending on the temperature of the dewatered thermally treated aqueous slurry of biomass fed into the cooling unit the filtrate of cold aqueous solution may not be sufficient to lower the temperature of the biomass to the desired temperature range. In that case additional cold liquid, preferably cold water, can be fed into the cooling unit. The additional cold liquid, preferably cold water, can be fed into the same feeding port as the filtrate of cold aqueous solution. It is, however, preferred to feed the additional cold liquid, preferably cold water, through a separate feeding port.

Depending on the temperature of the dewatered thermally treated aqueous slurry of biomass fed into the cooling unit the amount of filtrate of cold aqueous solution may exceed the amount of filtrate of cold aqueous solution necessary to lower the temperature of the biomass to the desired temperature range. In that case it is preferred to separate a part of the filtrate of hot aqueous solution and recycle said part into the pressurized prehydrolysis reactor unit. The other part of the filtrate of hot aqueous solution is then fed into the heat exchanger to produce the filtrate of cold aqueous solution.

Before feeding the filtrate of hot aqueous solution into the heat exchanger it can be subjected to a cleaning step in which e.g. impurities such as biomass residues or ash are separated. The filtrate of hot aqueous solution can also be subjected to a pH control by introducing pH buffer, if necessary, before being fed into the heat exchanger.

The dewatered thermally treated aqueous slurry of biomass is fed into a cooling unit for cooling by mixing the dewatered thermally treated aqueous slurry of biomass with a liquid which has a lower temperature than the dewatered thermally treated aqueous slurry of biomass.

Preferably the liquid for cooling the dewatered thermally treated aqueous slurry of biomass has a temperature in the range of from 10 to 40 °C, more preferably of 15 to 35 °C.

Preferably the filtrate of cold aqueous solution discharged from the heat exchanger is used as cooling liquid for the dewatered thermally treated aqueous slurry of biomass in the cooling unit.

For the case that the filtrate of cold aqueous solution is not sufficient for cooling the dewatered thermally treated aqueous slurry of biomass to the desired temperature range additional cold liquid, preferably cold water, is fed into the cooling unit.

The temperature of the additional cold liquid is preferably in the range of from 10 to 40 °C, more preferably of 15 to 35 °C.

The dewatered aqueous slurry of biomass is preferably cooled in the cooling unit to a temperature in the range of from 40 to 80 °C, more preferably of from 40 to 75 °C and most preferably of from 40 to 60 °C.

The cooling unit preferably includes a mixing entity for thoroughly mixing the dewatered aqueous slurry of biomass and the cooling liquid in a mixing zone of the cooling unit.

The cooling unit additionally comprises at least two feeding ports. One feeding port is used for feeding the dewatered aqueous slurry of biomass and the other feeding port is used for feeding the cooling liquid, preferably the filtrate of cold aqueous solution.

The cooling unit can also comprise additional feeding ports.

Into one optional additional feeding port additional cold liquid, preferably cold water, is fed, if necessary.

Into at least one other optional feeding port, which is/are located downstream of the mixing zone of the cooling unit, enzyme is introduced for hydrolyzing the cooled biomass.

Optionally, pH buffer is also fed into the cooling unit either at a feeding port in the mixing zone of at the same feeding port as the enzyme in order to adapt the pH of the cooled aqueous slurry of biomass to a suitable pH interval for enzymatic hydrolysis, such as a pH of between pH 4.7 and pH 5.3 or between pH 4.75 and pH 5.25 or around pH 5.

In another embodiment the cooled aqueous slurry of biomass is discharged from the cooling unit and fed into a separate hydrolysis unit in which it is treated with the enzyme.

The cooled aqueous slurry of biomass preferably has a temperature of from 40 to 80 °C, more preferably of from 40 to 75 °C and most preferably of from 40 to 60 °C.

Further, the cooled aqueous slurry of biomass preferably has a weight amount of biomass of from 15 to 25 wt%, more preferably of from 16 to 22 wt% and most preferably of from 17 to 20 wt%.

By enzymatic hydrolysis the carbohydrates such as cellulose in the biomass are hydrolysed to mono- or oligo-saccharides.

The method according to the invention can be in continuous or batch mode. For large scale industrial processes continuous operation is preferred.

### Device

The present invention further relates to for treating biomass comprising
- a biomass feeding system;
- a pressurized prehydrolysis reactor unit for thermally treating the biomass and for discharging a thermally treated aqueous slurry of biomass;
- a dewatering unit for separating the discharged thermally treated aqueous slurry of biomass into a dewatered thermally treated aqueous slurry of biomass and a filtrate of hot aqueous solution;
- a cooling unit;
wherein
the biomass feeding system is adapted to feed biomass to the pressurized prehydrolysis reactor unit and to feed the discharged thermally treated aqueous slurry of biomass from the pressurized prehydrolysis reactor unit through the dewatering unit to the cooling unit, and the cooling unit comprises
at least one feeding port for the dewatered thermally treated slurry of biomass;
at least one feeding port for a liquid which has a lower temperature than the dewatered thermally treated aqueous slurry of biomass;
means for mixing the dewatered thermally treated slurry of biomass and the liquid; and at least one feeding port for the enzyme for treating the cooled aqueous slurry of biomass downstream of the at least one feeding port for the dewatered thermally treated slurry of biomass and the at least one feeding port for a liquid which has a lower temperature than the dewatered thermally treated aqueous slurry of biomass; and
at least one discharge port for discharging the enzyme treated slurry of biomass from the cooling unit.

Preferably, the device according to the invention further comprises a heat exchanger unit for cooling the filtrate of a hot aqueous solution to produce a filtrate of a cold aqueous solution.

Still further, the device according to the invention preferably comprises a filtrate feeding system adapted to feed the filtrate of a hot aqueous solution from the dewatering unit to the heat exchanger unit and optionally to the pressurized prehydrolysis reactor unit, and to feed the filtrate of cold aqueous solution from the heat exchanger unit to the cooling unit.

The device as described above and below is suitable for the method according to the invention as described above or below.

### Brief description of the drawing

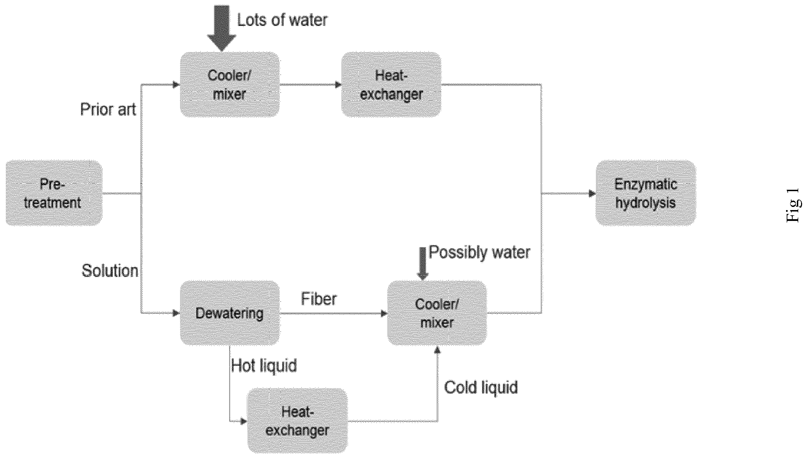

- Fig. 1: shows in the upper part a schematic plane of a device for executing a method for treating biomass as known in the art and
In the lower part a schematic plane of a device for executing a method for treating biomass according to the invention.

### Detailed description of the drawing

Fig 1 shows in the upper part a device for treating biomass as known in the art and in the lower part a schematic plane of a device for executing a method for treating biomass according to the invention.

Biomass is thermally treated a pressurized prehydrolysis reactor unit by heating and pressurizing with steam for a certain time. The biomass thereby can be heated by heating means of the pressurized prehydrolysis reactor unit (not shown) and pressurized by steam generation from steam generation means of the pressurized prehydrolysis reactor unit (not shown).

The thermally treated biomass can be discharged from the pressurized prehydrolysis reactor unit though steam explosion. The discharged thermally treated aqueous slurry of biomass discharged through steam explosion has a temperature, depending on the temperature in the pressurized prehydrolysis reactor unit, of from 160 °C to 220 °C, usually around 180 °C and a weight amount of biomass of 30 to 40 wt%. Alternatively, the thermally treated biomass can be diluted before it is discharged from the pressurized prehydrolysis reactor unit. In the latter case, biomass can either have a temperature below 100 °C, such as around 80 °C during discharging, wherein no flashing occurs during discharging, or the biomass can have a temperature above 100 °C, such as around 120 °C, during discharging and a flashing without steam explosion occurs during discharging. Due to the dilution the weight amount of the biomass in the discharged thermally treated aqueous slurry of biomass is significantly lower than after steam explosion. However, also the temperature is significantly lower. The discharged thermally treated aqueous slurry of biomass discharged through dilution discharge has a temperature of from 80 °C to 120 °C, usually around 80 °C or around 115 °C and a weight amount of biomass of 10 to 15 wt%.

In the device and method according to the prior art as shown in the upper part of Fig. 1 the discharged thermally treated aqueous slurry of biomass is cooled in two steps firstly by with mixing cold water in a cooling unit. In order to avoid a too high degree of dilution of the biomass below a desired weight amount for enzymatic hydrolysis of 17 to 20 wt% in the slurry of biomass, only limited amounts of cold water can be mixed into discharged thermally treated aqueous slurry of biomass which is not sufficient to cool the slurry to a temperature range suitable for downstream enzymatic hydrolysis of around 40 °C to 60 °C. For slurry discharged through dilution discharge the amount of cold water is thereby even more limited due to the already low weight amount of biomass in the slurry. Consequently, in order to further reduce the temperature of the aqueous slurry of biomass it is fed into a heat exchanger which cools the slurry without further diluting the biomass in the slurry. However, heat exchangers have the drawback of low cooling efficiency and can easily plug which can cause disruption of the process and additional costs.

In the device and method according to the present invention as shown in the lower part of Fig. 1 the discharged thermally treated aqueous slurry of biomass is firstly dewatered in a dewatering unit before fed into a cooling unit. In the dewatering unit a filtrate of hot aqueous solution is separated from the slurry of biomass to such an extent that the dewatered thermally treated aqueous slurry of biomass has a weight amount of biomass of 50 to 60 wt% after steam explosion discharge or, alternatively, of about 40 wt% after dilution discharge.

The filtrate of hot aqueous solution is cooled in a heat exchanger to a temperature of 15 to 35 °C and mixed in the cooling unit with the dewatered thermally treated aqueous slurry of biomass. In the case that the filtrate of cold aqueous solution is not sufficient to cool the slurry of biomass to the desired temperature of around 40 °C to 60 °C additional cold water can be added to the cooling unit.

Exceed filtrate of hot aqueous solution separated from the dewatering unit, which is not used for cooling the biomass slurry, can be separated before feeding into the heat exchanger and recycled into the pressurized prehydrolysis reactor unit (not shown).

The slurry of biomass cooled to around 40 °C to 60 °C has a weight amount of biomass of around 20 wt% which are the desired temperatures and weight amounts for ensuring optimum enzymatic hydrolysis conditions and lower energy consumption in the downstream processes as lower amounts of liquid have to be removed from the slurry.

## Claims

1. A method for treating biomass comprising
(a) Feeding biomass to a pressurized prehydrolysis reactor unit;
(b) Thermally treating the biomass in the pressurized prehydrolysis reactor unit;
(c) Discharging the thermally treated biomass from the pressurized prehydrolysis reactor unit to produce a discharged thermally treated aqueous slurry of biomass;
(d) Dewatering the discharged thermally treated aqueous slurry of biomass;
(e) Feeding the dewatered thermally treated aqueous slurry of biomass into a cooling unit;
(f) Cooling the dewatered thermally treated aqueous slurry of biomass by mixing the dewatered thermally treated aqueous slurry of biomass with a liquid which has a lower temperature than the dewatered thermally treated aqueous slurry of biomass in the cooling unit;
(g) Treating the cooled aqueous slurry of biomass with an enzyme.

2. The method according to claim 1, wherein the discharged thermally treated aqueous slurry of biomass is dewatered in a dewatering unit to separate a dewatered thermally treated aqueous slurry of biomass and a filtrate of a hot aqueous solution.

3. The method according to claim 2, wherein at least part of the filtrate of a hot aqueous solution is fed into a heat exchanger unit to produce a filtrate of a cold aqueous solution.

4. The method according to claim 3, wherein at least part of the filtrate of a cold aqueous solution is fed into the cooling unit of step (f) to cool the dewatered thermally treated aqueous slurry of biomass.

5. The method according to any one of claims 2 to 4, wherein a part of the filtrate of a hot aqueous solution is separated and fed into the pressurized prehydrolysis reactor unit.

6. The method according to any one of the preceding claims, wherein the thermally treated biomass has a temperature of from 120 °C to 220 °C and is discharged from the pressurized pretreatment unit through steam explosion to produce a discharged thermally treated aqueous slurry of biomass which has a weight amount of biomass of from 25 to 60 wt%.

7. The method according to claim 6, wherein the discharged thermally treated aqueous slurry of biomass is dewatered to a weight amount of biomass in the dewatered thermally treated aqueous slurry of biomass of from 30 to 60 wt%.

8. The method according to any one of the claims 1 to 5, wherein the thermally treated biomass has a temperature of from 50 °C to 140 °C and is discharged from the pressurized pretreatment unit through dilution discharge to produce a discharged thermally treated aqueous slurry of biomass which has a weight amount of biomass of from 5 to 25 wt%.

9. The method according to claim 8, wherein the discharged thermally treated aqueous slurry of biomass is dewatered to a weight amount of biomass in the dewatered thermally treated aqueous slurry of biomass of from 30 to 70 wt%.

10. The method according to any one of the preceding claims, wherein the liquid for cooling the dewatered thermally treated aqueous slurry of biomass in the cooling unit in step (f) has a temperature of from 10 to 40 °C.

11. The method according to any of the preceding claims, wherein dewatered thermally treated aqueous slurry of biomass is cooled in the cooling unit in step (f) to a temperature of 40-80 °C.

12. The method according to any of the preceding claims, wherein the cooled aqueous slurry of biomass in step (g) has a weight amount of biomass of from 15 to 25 wt%.

13. A device for treating biomass comprising
• a biomass feeding system;
• a pressurized prehydrolysis reactor unit for thermally treating the biomass and for discharging a thermally treated aqueous slurry of biomass;
• a dewatering unit for separating the discharged thermally treated aqueous slurry of biomass into a dewatered thermally treated aqueous slurry of biomass and a filtrate of hot aqueous solution;
• a cooling unit;
wherein
the biomass feeding system is adapted to feed biomass to the pressurized prehydrolysis reactor unit and to feed the discharged thermally treated aqueous slurry of biomass from the pressurized prehydrolysis reactor unit through the dewatering unit to the cooling unit, and
the cooling unit comprises
at least one feeding port for the dewatered thermally treated slurry of biomass; at least one feeding port for a liquid which has a lower temperature than the dewatered thermally treated aqueous slurry of biomass;
means for mixing the dewatered thermally treated slurry of biomass and the liquid; and
at least one feeding port for the enzyme for treating the cooled aqueous slurry of biomass downstream of the at least one feeding port for the dewatered thermally treated slurry of biomass and the at least one feeding port for a liquid which has a lower temperature than the dewatered thermally treated aqueous slurry of biomass; and
at least one discharge port for discharging the enzyme treated slurry of biomass from the cooling unit.

14. The device for treating biomass according to claim 13 further comprising a heat exchanger unit for cooling the filtrate of a hot aqueous solution to produce a filtrate of a cold aqueous solution.

15. The device for treating biomass according to claim 14 further comprising a filtrate feeding system adapted to feed the filtrate of a hot aqueous solution from the dewatering unit to the heat exchanger unit and optionally to the pressurized prehydrolysis reactor unit, and to feed the filtrate of cold aqueous solution from the heat exchanger unit to the cooling unit.

## Patentansprüche

1. Ein Verfahren zur Behandlung von Biomasse, umfassend
(a) Einspeisung von Biomasse in eine unter Druck stehende Reaktoreinheit zur Vorhydrolyse;
(b) Thermische Behandlung der Biomasse in der unter Druck stehenden Reaktoreinheit zur Vorhydrolyse;
(c) Austragen der thermisch behandelten Biomasse aus der unter Druck stehenden Reaktoreinheit zur Vorhydrolyse, um einen ausgetragenen thermisch behandelten wässrigen Schlamm aus Biomasse zu erzeugen;
(d) Entwässerung des ausgetragenen thermisch behandelten wässrigen Schlamms aus Biomasse;
(e) Einspeisung des entwässerten thermisch behandelten wässrigen Schlamms aus Biomasse in eine Kühleinheit;
(f) Kühlen des entwässerten thermisch behandelten wässrigen Schlamms der Biomasse durch Mischen des entwässerten thermisch behandelten wässrigen Schlamms der Biomasse mit einer Flüssigkeit, die eine niedrigere Temperatur als der entwässerte thermisch behandelte wässrige Schlamm der Biomasse in der Kühleinheit hat;
(g) Behandlung des gekühlten wässrigen Schlamms der Biomasse mit einem Enzym.

2. Das Verfahren nach Anspruch 1, wobei der ausgetragene thermisch behandelte wässrige Schlamm aus Biomasse in einer Entwässerungseinheit entwässert wird, um einen entwässerten thermisch behandelten wässrigen Schlamm aus Biomasse und ein Filtrat einer heißen wässrigen Lösung zu trennen.

3. Das Verfahren nach Anspruch 2, wobei mindestens ein Teil des Filtrats einer heißen wässrigen Lösung in eine Einheit zum Wärmeaustausch geleitet wird, um ein Filtrat einer kalten wässrigen Lösung zu erzeugen.

4. Das Verfahren nach Anspruch 3, wobei zumindest ein Teil des Filtrats einer kalten wässrigen Lösung in die Kühleinheit von Schritt (f) eingespeist wird, um den entwässerten thermisch behandelten wässrigen Schlamm der Biomasse zu kühlen.

5. Das Verfahren nach einem der Ansprüche 2 bis 4, wobei ein Teil des Filtrats einer heißen wässrigen Lösung abgetrennt und in die unter Druck stehende Reaktoreinheit zur Vorhydrolyse eingespeist wird.

6. Das Verfahren nach einem der vorhergehenden Ansprüche, wobei die thermisch behandelte Biomasse eine Temperatur von 120 °C bis 220 °C hat und aus der unter Druck stehenden Vorbehandlungseinheit durch Dampfexplosion ausgetragen wird, um einen ausgetragenen thermisch behandelten wässrigen Schlamm von Biomasse zu erzeugen, der einen Gewichtsanteil an Biomasse von 25 bis 60 Gew.-% hat.

7. Das Verfahren nach Anspruch 6, wobei der ausgetragene thermisch behandelte wässrige Schlamm aus Biomasse auf eine Gewichtsmenge an Biomasse in dem entwässerten thermisch behandelten wässrigen Schlamm aus Biomasse von 30 bis 60 Gew.-% entwässert wird.

8. Das Verfahren nach einem der Ansprüche 1 bis 5, wobei die thermisch behandelte Biomasse eine Temperatur von 50 °C bis 140 °C aufweist und aus der unter Druck stehenden Vorbehandlungseinheit durch Verdünnungsaustrag ausgetragen wird, um einen ausgetragenen thermisch behandelten wässrigen Schlamm von Biomasse zu erzeugen, der eine Gewichtsmenge an Biomasse von 5 bis 25 Gew.-% aufweist.

9. Das Verfahren nach Anspruch 8, wobei der ausgetragene thermisch behandelte wässrige Schlamm aus Biomasse auf eine Gewichtsmenge an Biomasse in dem entwässerten thermisch behandelten wässrigen Schlamm aus Biomasse von 30 bis 70 Gew.-% entwässert wird.

10. Das Verfahren nach einem der vorhergehenden Ansprüche, wobei die Flüssigkeit zur Kühlung des entwässerten thermisch behandelten wässrigen Schlamms aus Biomasse in der Kühleinheit in Schritt (f) eine Temperatur von 10 bis 40 °C aufweist.

11. Das Verfahren nach einem der vorhergehenden Ansprüche, bei dem der entwässerte thermisch behandelte wässrige Schlamm der Biomasse in der Kühleinheit in Schritt (f) auf eine Temperatur von 40-80 °C abgekühlt wird.

12. Das Verfahren nach einem der vorhergehenden Ansprüche, wobei der gekühlte wässrige Schlamm der Biomasse in Schritt (g) eine Gewichtsmenge an Biomasse von 15 bis 25 Gew.-% aufweist.

13. Eine Vorrichtung zur Behandlung von Biomasse mit
• Eine Beschickung für Biomasse;
• eine unter Druck stehende Reaktoreinheit zur Vorhydrolyse zur thermischen Behandlung der Biomasse und zur Entnahme eines thermisch behandelten wässrigen Schlamms der Biomasse;
• eine Entwässerungseinheit zum Trennen des ausgetragenen thermisch behandelten wässrigen Schlamms aus Biomasse in einen entwässerten thermisch behandelten wässrigen Schlamm aus Biomasse und ein Filtrat einer heißen wässrigen Lösung;
• ein Kühleinheit;
wobei
die Beschickung für Biomasse so ausgelegt ist, dass sie der unter Druck stehenden Reaktoreinheit zur Vorhydrolyse Biomasse zuführt und den ausgetragenen thermisch behandelten wässrigen Schlamm der Biomasse aus der unter Druck stehenden Reaktoreinheit zur Vorhydrolyse durch die Entwässerungseinheit zur Kühleinheit leitet, und
die Kühleinheit umfasst
mindestens eine Zufuhröffnung für den entwässerten thermisch behandelten Schlamm aus Biomasse;
mindestens eine Zuführöffnung für eine Flüssigkeit, die eine niedrigere Temperatur hat als der entwässerte thermisch behandelte wässrige Schlamm aus Biomasse;
Mittel zum Mischen des entwässerten thermisch behandelten Schlamms aus Biomasse und der Flüssigkeit; und
mindestens eine Zuführöffnung für das Enzym zur Behandlung des gekühlten wässrigen Schlamms der Biomasse stromabwärts der mindestens einen Zuführöffnung für den entwässerten thermisch behandelten Schlamm der Biomasse und der mindestens einen Zuführöffnung für eine Flüssigkeit, die eine niedrigere Temperatur als der entwässerte thermisch behandelte wässrige Schlamm der Biomasse hat; und
mindestens eine Austragsöffnung zum Austragen des mit Enzymen behandelten Schlamms der Biomasse aus der Kühleinheit.

14. Die Vorrichtung zur Behandlung von Biomasse nach Anspruch 13 ferner umfassend eine Einheit zum Wärmeaustausch zum Kühlen des Filtrats einer heißen wässrigen Lösung, um ein Filtrat einer kalten wässrigen Lösung zu erzeugen.

15. Die Vorrichtung zur Behandlung von Biomasse nach Anspruch 14, die ferner eine Zuführung für Filtrat umfasst, die so beschaffen ist, dass es das Filtrat einer heißen wässrigen Lösung von der Entwässerungseinheit der Einheit zum Wärmeaustausch und gegebenenfalls der unter Druck stehenden Reaktoreinheit zur Vorhydrolyse zuführt und das Filtrat der kalten wässrigen Lösung von der Einheit zum Wärmeaustausch der Kühleinheit zuführt.

## Revendications

1. Procédé pour traiter de la biomasse comprenant
(a) fournir de la biomasse à une unité de réacteur à préhydrolyse mise sous pression ;
(b) traiter thermiquement la biomasse dans l'unité de réacteur à préhydrolyse mise sous pression ;
(c) décharger la biomasse traitée thermiquement de l'unité de réacteur à préhydrolyse mise sous pression pour produire une boue de biomasse aqueuse traitée thermiquement déchargée ;
(d) déshydrater la boue de biomasse aqueuse traitée thermiquement déchargée ;
(e) fournir la boue de biomasse aqueuse traitée thermiquement déshydratée à une unité de refroidissement ;
(f) refroidir la boue de biomasse aqueuse traitée thermiquement déshydratée en mélangeant la boue de biomasse aqueuse traitée thermiquement déshydratée avec un liquide qui a une température plus basse que la boue de biomasse aqueuse traitée thermiquement déshydratée dans l'unité de refroidissement ;
(g) traiter la boue de biomasse aqueuse refroidie avec une enzyme.

2. Procédé selon la revendication 1, dans lequel la boue de biomasse aqueuse traitée thermiquement déchargée est déshydratée dans une unité de déshydratation pour séparer une boue de biomasse aqueuse traitée thermiquement déshydratée et un filtrat d'une solution aqueuse chaude.

3. Procédé selon la revendication 2, dans lequel une partie au moins du filtrat d'une solution aqueuse chaude est fournie à une unité d'échangeur de chaleur pour produire un filtrat d'une solution aqueuse froide.

4. Procédé selon la revendication 3, dans lequel une partie au moins du filtrat d'une solution aqueuse froide est fournie à l'unité de refroidissement de l'étape (f) pour refroidir la boue de biomasse aqueuse traitée thermiquement déshydratée.

5. Procédé selon l'une quelconque des revendications 2 à 4, dans lequel une partie au moins du filtrat d'une solution aqueuse chaude est séparée et conduite dans l'unité de réacteur à préhydrolyse mise sous pression.

6. Procédé selon l'une quelconque des revendications précédentes, dans lequel la biomasse traitée thermiquement a une température de 120°C à 220°C et est déchargée de l'unité de prétraitement mise sous pression par explosion de vapeur pour produire une boue de biomasse aqueuse traitée thermiquement déchargée qui a une quantité en poids de biomasse de 25 à 60 % en poids.

7. Procédé selon la revendication 6, dans lequel la boue de biomasse aqueuse traitée thermiquement déchargée est déshydratée à une quantité en poids de biomasse dans la boue de biomasse aqueuse traitée thermiquement déshydratée de 30 à 60 % en poids.

8. Procédé selon l'une quelconque des revendications 1 à 5, dans lequel la biomasse traitée thermiquement a une température de 50°C à 140°C et est déchargée de l'unité de prétraitement mise sous pression par décharge à dilution pour produire une boue de biomasse aqueuse traitée thermiquement déchargée qui a une quantité en poids de biomasse de 5 à 25 % en poids.

9. Procédé selon la revendication 8, dans lequel la boue de biomasse aqueuse traitée thermiquement déchargée est déshydratée à une quantité en poids de biomasse dans la boue de biomasse aqueuse traitée thermiquement déshydratée de 30 à 70 % en poids.

10. Procédé selon l'une quelconque des revendications précédentes, dans lequel le liquide pour refroidir la boue de biomasse aqueuse traitée thermiquement déshydratée a dans l'unité de refroidissement de l'étape (f) une température de 10 à 40°C.

11. Procédé selon l'une quelconque des revendications précédentes, dans lequel la boue de biomasse aqueuse traitée thermiquement déshydratée est refroidie dans l'unité de refroidissement de l'étape (f) à une température de 40-80°C.

12. Procédé selon l'une quelconque des revendications précédentes, dans lequel la boue de biomasse aqueuse refroidie a dans l'étape (g) une quantité en poids de biomasse de 15 à 25 % en poids.

13. Dispositif pour traiter de la biomasse comprenant
• un système de fourniture de biomasse ;
• une unité de réacteur à préhydrolyse mise sous pression pour traiter thermiquement la biomasse et pour décharger une boue de biomasse aqueuse traitée thermiquement ;
• une unité de déshydratation pour séparer la boue de biomasse aqueuse traitée thermiquement déchargée en une boue de biomasse aqueuse traitée thermiquement déshydratée et un filtrat d'une solution aqueuse chaude ;
• une unité de refroidissement ;
dans lequel
le système de fourniture de biomasse est adapté pour fournir de la biomasse à l'unité de réacteur à préhydrolyse mise sous pression et pour fournir la boue de biomasse aqueuse traitée thermiquement déchargée à l'unité de réacteur à préhydrolyse mise sous pression, à travers l'unité de déshydratation, à l'unité de refroidissement, et l'unité de refroidissement comprend
au moins un orifice de fourniture pour la boue de biomasse traitée thermiquement déshydratée ;
au moins un orifice de fourniture pour un liquide qui a une température plus basse que la boue de biomasse aqueuse traitée thermiquement déshydratée ;
des moyens pour mélanger la boue de biomasse traitée thermiquement déshydratée et le liquide ; et
au moins un orifice de fourniture pour l'enzyme pour traiter la boue de biomasse aqueuse refroidie en aval du au moins un orifice de fourniture pour la boue de biomasse traitée thermiquement déshydratée et du au moins un orifice de fourniture pour un liquide qui a une température plus basse que la boue de biomasse aqueuse traitée thermiquement déshydratée ; et
au moins un orifice de décharge pour décharger de l'unité de refroidissement la boue de biomasse traitée par enzyme.

14. Dispositif pour traiter de la biomasse selon la revendication 13 comprenant en outre une unité d'échangeur de chaleur pour refroidir le filtrat d'une solution aqueuse chaude afin de produire un filtrat d'une solution aqueuse froide.

15. Dispositif pour traiter de la biomasse selon la revendication 14 comprenant en outre un système de fourniture de filtrat adapté pour conduire le filtrat d'une solution aqueuse chaude de l'unité de déshydratation à l'unité d'échangeur de chaleur et optionnellement à l'unité de réacteur à préhydrolyse mise sous pression, et pour conduire le filtrat de solution aqueuse froide de l'unité d'échangeur de chaleur à l'unité de refroidissement.
